(19) **Europäisches Patentamt / European Patent Office / Office européen des brevets**

(11) **EP 4 019 939 B1**

(12) **EUROPEAN PATENT SPECIFICATION**

(45) Date of publication and mention
of the grant of the patent:
**29.04.2026 Bulletin 2026/18**

(21) Application number: **21212867.2**

(22) Date of filing: **07.12.2021**

(51) International Patent Classification (IPC):
**G01N 21/05** (2006.01)     **G01N 33/18** (2006.01)
**G01N 21/03** (2006.01)

(52) Cooperative Patent Classification (CPC):
**G01N 21/05; G01N 33/1893;** G01N 2021/0378;
G01N 2021/052; G01N 2201/08

(54) **SYSTEM FOR MEASURING CONCENTRATION OF UREA IN A SOLUTION WITH WATER**

SYSTEM ZUR MESSUNG DER HARNSTOFFKONZENTRATION IN EINER LÖSUNG MIT WASSER

SYSTÈME DE MESURE DE LA CONCENTRATION D'URÉE DANS UNE SOLUTION AVEC DE L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2020 IT 202000032162**

(43) Date of publication of application:
**29.06.2022 Bulletin 2022/26**

(73) Proprietor: **Ecomec Impianti S.r.l.**
**10024 Moncalieri (Torino) (IT)**

(72) Inventor: **SALVATORE, Osvaldo**
**I-10024 Moncalieri (Torino) (IT)**

(74) Representative: **Buzzi, Notaro & Antonielli d'Oulx S.p.A.**
**Corso Vittorio Emanuele II, 6**
**10123 Torino (IT)**

(56) References cited:
**EP-A1- 2 058 682         WO-A1-2008/107336**
**WO-A1-2012/170743     US-A- 3 513 319**
**US-A1- 2002 102 183**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present disclosure relates to a system for measuring the concentration of urea in solution with water.

Systems of this type are today used in the motor-vehicle sector in association with devices for the reduction of the emissions of nitrogen oxide by way of a solution of urea in water. The solution in question operates by releasing ammonia, which combines with the nitrogen oxides to obtain free nitrogen and water.

WO2012/170743A1, US3513319A, US2002/102183A1, EP2058682A1 and WO2008/107336A1 disclose prior art that may be of interest as technical background.

A known system for measuring the concentration of urea in solution with water envisages measurement of the losses of intensity of a light beam that is conveyed along an optical fibre immersed within the solution to be tested (the optical fibre is thus made up of a core of transparent material and the cladding constituted by the solution to be measured). Estimation of the concentration of urea is based upon the fact that the value of the losses of intensity of the light beam depends upon the index of refraction of the solution, which in turn is a function of the concentration of urea.

The above system presents a series of drawbacks, amongst which it is here pointed out, in particular, the fact that the precision of the system may be jeopardized by ageing of the source of the light beam, which can cause a lowering of intensity of the light beam emitted by the source and hence cause erroneous determinations of the value of the losses of intensity of the light beam along the optical fibre.

To overcome the above drawback, solutions have been proposed that measure also the intensity of the light beam emitted by the source. These solutions are, however, somewhat complex in so far as they necessitate two lines for measuring the light intensity that require two distinct lines of amplification of the light signal and two different photodiodes with different dark current and different pull-up resistance.

On the other hand, errors in the determination of the losses of intensity of the light beam along the optical fibre can be generated also as a result of imprecise positioning, during assembly of the system, between the source of the light beam and the optical fibre.

As a whole, the known measurement system discussed above requires a complicated structure and somewhat complex control modalities, proving, in any case, far from reliable.

In this context, the object of the present invention is to provide a measurement system that will be improved as compared to the prior art, and in particular will overcome the aforesaid drawbacks.

The above object is achieved by way of a measurement system as defined in independent claim 1.

Preferred embodiments are defined in the dependent claims.

Further characteristics and advantages of the present invention will emerge clearly from the ensuing description and from the annexed drawings, wherein:

- Figure 1 is a schematic illustration of a preferred embodiment of the measurement system described herein;
- Figure 2 illustrates a further embodiment of the system described herein;
- Figure 3 regards a graph of experimental data of a specific application of the solution described herein;
- Figure 4 regards a further graph of experimental data of a specific application of the solution described herein;
- Figure 5 is a schematic illustration of a further embodiment of the system described herein; and
- Figure 6 is a schematic illustration of a further embodiment of the system described herein.

In the ensuing description various specific details are illustrated aimed at enabling an in-depth understanding of the embodiments. The references used herein are only for convenience and hence do not define the sphere of protection or the scope of the embodiments.

In general, the system described herein for measuring the concentration of urea in solution with water comprises:

- an emitter 2 of a beam of electromagnetic waves;
- a receiver 4 of the beam of electromagnetic waves;
- a duct 6 provided for containing the solution of urea and water;
- a first optical element 8 configured for optically coupling a first end of the duct 6 with the emitter 2;
- a second optical element 12 configured for optically coupling a second end 6B, opposite to the first end 6A, of the duct 6 with the receiver 4; and
- a control unit 20 configured for determining the concentration of urea in the solution contained in the duct 6 as a function of a signal generated by the receiver 4.

The receiver 4 is configured for detecting the variation in time of the intensity of the beam of electromagnetic waves, and the control unit 20 is configured for determining the concentration of urea in the solution with water as a function of the variation in unit time of the intensity of the beam of electromagnetic waves received by the receiver 4.

The receiver 4 is subjected to the beam of electromagnetic waves for a pre-set exposure time. For instance, the receiver 4 comprises a shutter designed to be operated for the purpose of defining an exposure time. The exposure time is defined for carrying out detection of the beam of electromagnetic waves in pre-set conditions so as to guarantee reliability and repeatability of the measurements, and can be selected on the basis of the specifications of the system, amongst which the type of emitter 2 and receiver 4, as well as their mode of operation.

It will be noted that the beam of electromagnetic waves that from the emitter 2 reaches the receiver 4 - following the optical path through the first optical element 8, the duct 6, and the second optical element 12 - is in actual fact constituted by a plurality of electromagnetic waves that have different modes of propagation.

The range of the modes of propagation in the light duct described is a function of the concentration of the urea in water and of the index of refraction of the material constituting the duct.

As a result of the different modes of propagation, these electromagnetic waves will follow distinct paths and will present different losses of intensity along the optical path referred to above.

From the standpoint of detection by the receiver 4, this means that this will determine an intensity of the beam of electromagnetic waves variable in time.

In this context, the present applicant has been able to find experimentally that the variation in unit time of the intensity of the beam of electromagnetic waves received by the receiver 4 is characteristic of the degree of concentration of urea in the solution.

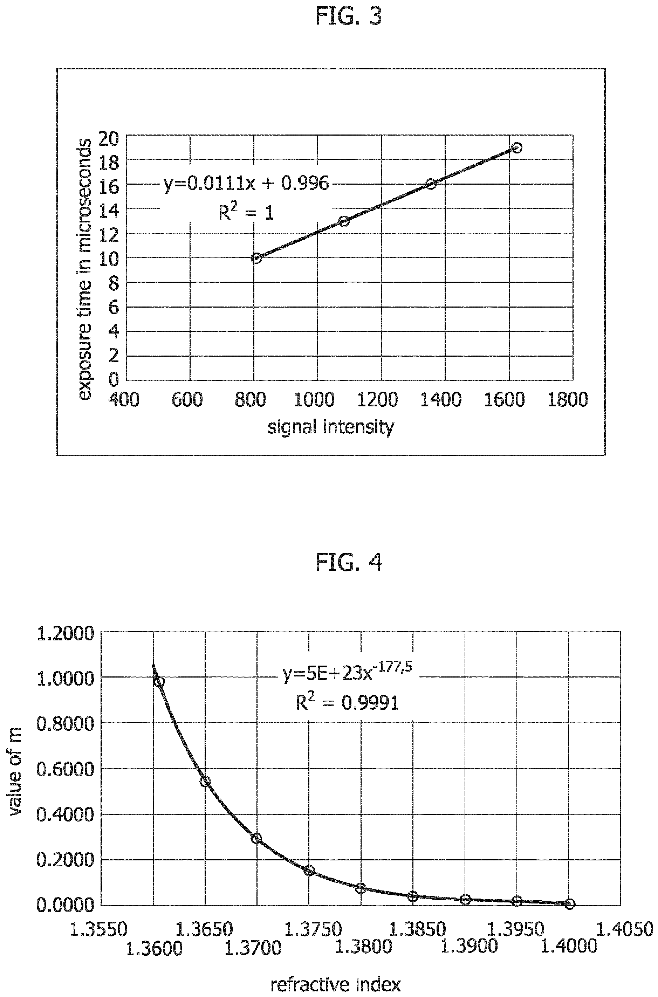

For a given concentration of urea, the variation in unit time of the intensity of the beam of electromagnetic waves has proven, in fact, substantially constant. In this connection, Figure 3 illustrates an example of plot of the intensity of the beam of electromagnetic waves detected for a given concentration of urea.

In view of the foregoing, the variation in unit time of the intensity of the beam of electromagnetic waves received by the receiver 4 can be related via a function to the concentration of the solution.

In an example of application, the present applicant has identified the following function:

$$Y = A \cdot X^{-k}$$

where Y is the concentration of the solution, X is the variation in unit time of the intensity of the beam of electromagnetic waves, and A and K are two parameters the values of which depend upon the physical and geometrical characteristics of the specific system used.

In general, the relation between the concentration of urea and the variation of intensity of the beam of electromagnetic waves may be determined experimentally for any specific application. Figure 4 illustrates an example of function obtained for a specific application; the values of m represented correspond to the variation of the intensity of the beam of electromagnetic waves.

The relation thus obtained can be saved in a memory unit (not illustrated).

The control unit 20 can be prearranged for determining the variation in unit time of the intensity of the beam of electromagnetic waves on the basis of the signals generated by the receiver 4, and for determining the concentration of urea using the relation stored in the aforesaid memory unit.

The person skilled in of the sector will now understand that the variation in time of the intensity of the beam of electromagnetic waves is in itself independent of the nominal or real value of the intensity of the beam of waves emitted by the emitter, so that any possible ageing of the source of the beam, drifts of the amplification lines, or else imprecisions in assembly of the system do not vitiate in any way the measurement made by the system.

The drawbacks of the prior art discussed at the start are consequently overcome.

In a preferred embodiment, the system described herein comprises a ToF (Time-of-Flight) sensor, for instance of a commercially available type, which has the emitter 2 and the receiver 4 integrated together in one and the same measurement device. The receiver and the emitter are synchronized so that for each measurement made the device is able to provide a summation of the amplitudes of all the transmission modes received during exposure and a function correlated to the measurement of all the distances covered by the transmission modes themselves.

The experiments conducted have shown that the correlation of the concentration of urea with the summation of the amplitudes is precise and repeatable.

The system described herein envisages the use of a duct containing the solution of urea and water to be measured in order to define the optical path of the beam of electromagnetic waves from the emitter 2 to the receiver 4.

Through the choice of the configuration of the duct as a function of the needs of the specific applications, it is possible to define the optical path in such a way as to guarantee measurements that are precise and stable in time.

In preferred embodiments, as in the one illustrated, the emitter 2 is a LED or VCSEL (Vertical-Cavity Surface-Emitting Laser) source and has a main direction of emission oriented in a direction parallel to a reference direction R of the system. Preferably, the beam of electromagnetic waves emitted by the emitter 2 has a wavelength that is optimized as a function of the curve of spectral sensitivity of the receiver and of the absorption of the medium (solution).

In preferred embodiments, as in the one illustrated, the receiver 4 is positioned alongside the emitter 2 and is oriented so that the axis orthogonal to its surface 4I of detection is also parallel to the reference direction R. The receiver 4 may, for example, be a common photoelectric sensor.

Preferably, the emitter 2 emits light modulated in time (with rectangular pulses or continuously). As has been seen, the receiver 4 is used for detecting the intensity of the beam of light received, in particular enabling analysis of its variation in time.

In preferred embodiments, as in the one illustrated, the duct 6 extends along a curvilinear path that subtends a pre-set length L of the duct 6 and at the same time defines a dimension H of the duct in the reference direction R considerably smaller than the length L.

In preferred embodiments, as in the one illustrated, the path followed by the duct 6 has a generically helical profile that develops about an axis orthogonal to the direction R.

In particular, in the example illustrated, starting from the emitter 2, the duct 6 has a first rectilinear stretch 6I, which defines the end 6A of the duct, a second stretch 6II, having a generically helical profile, and a third, rectilinear, stretch 6III, which defines the opposite end 6B of the duct.

The two rectilinear stretches 6I and 6III are both oriented in a direction parallel to the reference direction R.

In preferred embodiments, as in the one illustrated, the two optical elements 8, 12 have a cylindrical conformation and are inserted within the stretches 6I and 6III, respectively, setting themselves coaxial thereto. The two optical elements 8 and 12 may be two optical fibres, micro-lenses of a selfoc type for collimation or focusing or simple transparent cylinders, for example made of transparent polysulphone or Duran G glass.

The ends of the portions of the two elements 8, 12 that exit from the two stretches 6I, 6III of the duct face the emitter 2 and the receiver 4, respectively.

The duct 6 is configured to be able to operate, once it is filled with the solution of water and urea, as an optical fibre designed to conduct a beam of electromagnetic waves from one end to the other of the fibre. Preferably, the duct 6 is constituted by a tube having a circular cross section and constituted by transparent or translucid walls. Preferably, the duct 6 is made of PTFE, PTFE FEP, PFA as examples of materials that are chemically inert in regard to the aqueous solutions undergoing measurement and with low refractive index. Clearly, the material of which the duct 6 is made has a refractive index lower than that of the solvent (water = 0% urea concentration) of the solution to be measured, so that the duct 6 containing the solution can operate as light guide. In alternative embodiments, the duct 6 can follow paths of a type different from the one illustrated above, for example, a U-shaped path, a spiral path, a serpentine path, etc., in any case always characterized in that its length L is substantially greater than the effective dimension of the duct 6 in the reference direction R. Moreover, also cross sections different from the circular one may be adopted.

The dimensions L and H may clearly vary on the basis of the requirements of the specific applications. On the one hand, the dimension H may in particular depend upon the space available for the duct 6 in the site where the system is installed. On the other hand, the pre-set length L may instead be advantageously selected for determining times of flight of the optical path by the beam of electromagnetic waves that are sufficiently long as to facilitate detection by the receiver 4 and analysis of the data by the control unit 20.

On the other hand, it will be noted that the system may envisage optical elements additional to the ones illustrated to collimate, focus, or treat the beam in some other way so as to favour its transport along the duct 6 and facilitate detection thereof by the receiver 4.

In order to make it possible to fill the duct 6 with the solution to be tested, the duct 6 itself has an inlet 6C and an outlet 6D for the solution, which are located in intermediate positions between the two ends 6A and 6B thereof; in particular, the inlet 6C is positioned closer to the end 6A, whereas the outlet 6D is positioned closer to the end 6B.

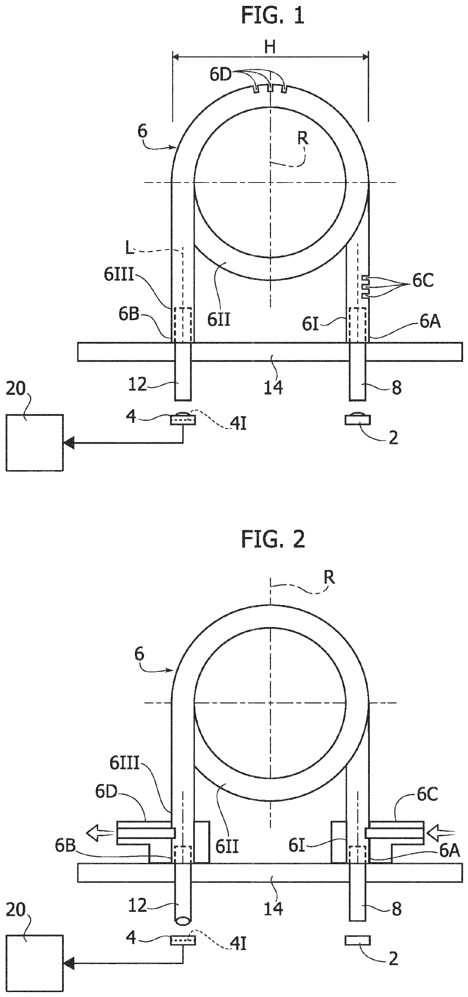

Circulation of the solution through the duct 6 may be determined by a natural motion that is set up within the tank containing the solution, as in the embodiment of Figure 1, or else may be a forced circulation as in the embodiment of Figure 2.

With reference to Figure 1, the system 10 is directly inserted within the tank that is to contain the solution of water and urea to be fed to the NOX-reduction device. The system comprises a support 14, preferably in the form of a plate, which is to define a portion of the bottom of the tank, and mounted on which are the emitter 2, the receiver 4, the duct 6, and the two optical elements 8, 12.

In particular, the duct 6 is mounted on the side of the support 14 that faces the inside of the tank, whereas the emitter 2 and the receiver 4 are mounted on the opposite side. The two optical elements 8, 12 traverse the support 14, at respective openings provided thereon, optically coupling the ends 6A, 6B of the duct 6 with the emitter 2 and the receiver 4, respectively.

It will be noted that the support 14 creates a barrier that separates the area of the tank immersed in the solution, located in which is the duct 6 itself, from the emitter 2 and the receiver 4.

In this embodiment, the inlet 6C and the outlet 6D are constituted by simple openings that set in connection the inside of the duct with the outside. In the embodiment illustrated, the inlet 6C is positioned in the proximity of the end 6A of the duct, substantially at the end of the stretch 6I. The outlet 6D is, instead, positioned at the start of the rectilinear stretch 6III, substantially on the top of the duct 6 (with respect to the support 14). The difference in height from the support 14 between the inlet 6C and the outlet 6D brings about, as a result of the hydrostatic forces, a natural circulation of the solution of water and urea along the duct 6, from the inlet 6C to the outlet 6D.

This natural motion of the solution of water and urea guarantees total filling of the duct 6 by the solution at the moment when the corresponding tank is filled, and moreover favours exit of possible air bubbles from the duct 6, which could alter the measurements made by the system.

Once again with reference to the embodiment of Figure 1, the person skilled in the sector will understand that the system 10 thus obtained is able to operate also as fall-back level sensor for the solution of water and urea contained in the corresponding tank. In fact, lowering of the free surface of the solution contained in the tank underneath the outlet 6D will cause inlet of air into the duct 6 and, consequently, the intensity of the beam of electromagnetic waves transmitted to the receiver 4 will be drastically reduced or even vanish completely.

The control unit 12 may be configured for detecting such a condition on the basis of the signals coming from the receiver 4.

With reference now to the embodiment of Figure 2, this differs form the previous one mainly in that the system is not inserted inside the tank that is to contain the solution of water and urea, but is separated from this, it being preferably located in an intermediate position between the tank and the NOX-reduction device fed thereby.

According to this embodiment, the inlet 6C and the outlet 6D are constituted by hydraulic connectors configured for being connected to the line that feeds the solution of water and urea from the tank to the NOX-reduction device. Preferably, in this embodiment, the inlet 6C and the outlet 6D are positioned in the proximity of the ends 6A and 6B of the duct 6, respectively.

In this case, the duct 6 is traversed by a flow of the solution of water and urea that is fed by an, external, pressure-generating device. According to an advantageous application, the system can be integrated within the line for supply of the solution of water and urea so as to exploit the pump of the supply line itself to generate a flow of solution through the duct 6. Alternatively, the system may be positioned at the bottom of the tank of the solution of water and urea.

For both the embodiments of Figure 1 and Figure 2, the system envisages gasket elements - not illustrated - for sealing the parts that are to receive the solution with respect to the remaining parts of the system.

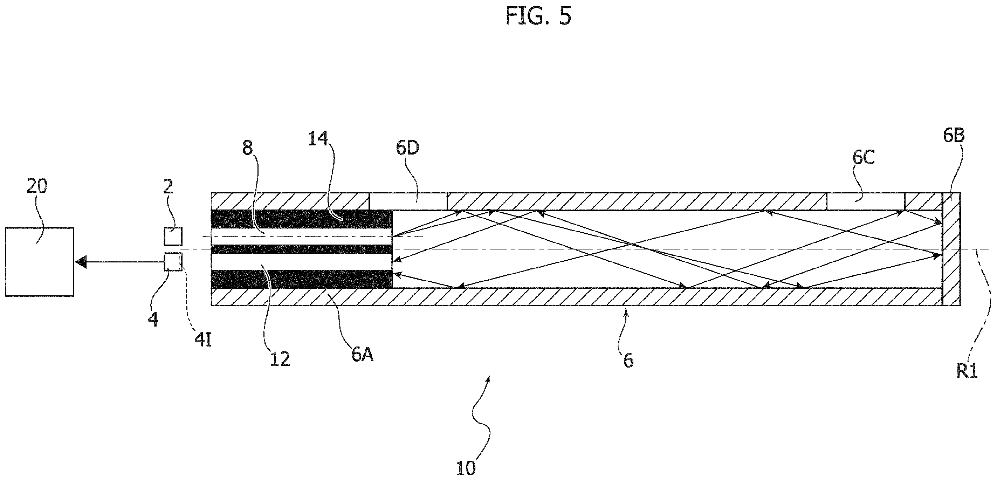

With reference now to the embodiment of Figure 5 (for simplicity of treatment the components in common with those of the previous embodiments are designated by the same reference numbers used above), this differs from the two embodiments of Figures 1 and 2 in that the duct 6 is rectilinear and the ends 6A and 6B are, hence, located along one and the same axis R1.

The emitter 2 has a main direction of emission oriented in a direction parallel to the axis R1. The receiver 4 is positioned alongside the emitter 2 and is oriented so that the axis orthogonal to its surface 4I of detection is also parallel to the axis R1.

Moreover, in this embodiment, the emitter 2 and the receiver 4 are both operatively connected to the end 6A of the duct through the optical elements 8, 12, in a way similar to what has been described above for the previous embodiments. On the other hand, the opposite end 6B of the duct 6 is closed to reflect back the electromagnetic waves emitted by the emitter 2 and coming from the end 6A. The inlet 6C and the outlet 6D are, instead, located once again in positions comprised between the two ends 6A, 6B and, in particular, the inlet 6C is set in the proximity of the end 6A, while the outlet 6D is set in the proximity of the end 6B.

It will be noted that in this embodiment the beam of electromagnetic waves emitted by the emitter 2 follows an optical path characterized by a forward stretch and a return stretch that both develop along the duct 6, the forward stretch extending from the first end 6A to the second end 6B, the return stretch extending from the second end 6B to the first end 6A. The optical path passes a first time through the end 6A, in the forward stretch, traversing the optical element 8, and passes a second time through the same end 6A, in the return stretch, traversing, instead, the optical element 12.

Apart from the differences described above, determination of the concentration of urea in the solution is obtained with the same modalities as those described previously. As envisaged for the previous embodiments, the system 10 according to this further embodiment may be inserted directly within the tank containing the solution of water and urea. Likewise, circulation of the solution through the duct 6 can be determined by a natural motion, as in the embodiment of Figure 1, or else via forced circulation, as in the embodiment of Figure 2.

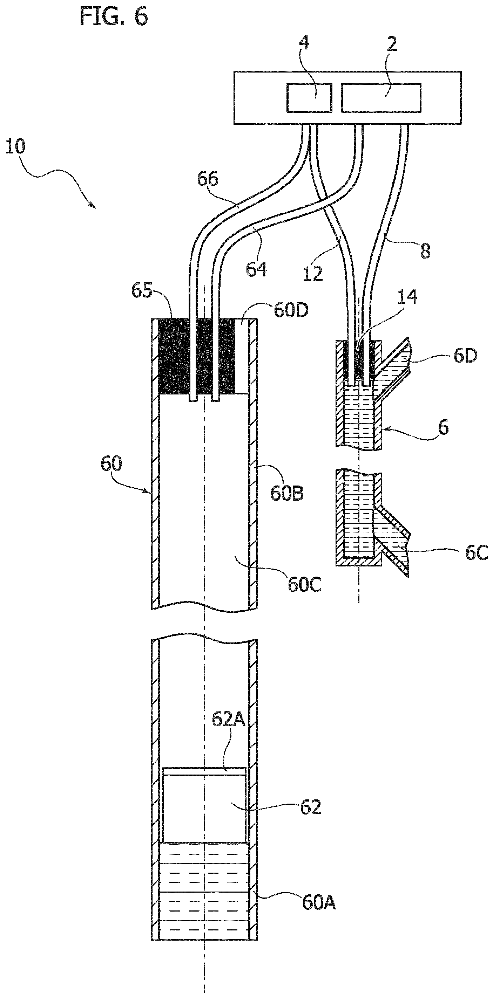

Figure 6 illustrates a further embodiment of the system described herein, in which the system 10, provided according to the teachings described above with reference to the embodiment of Figure 5, is moreover configured to measure the level of the solution of water and urea in the tank. For this purpose, the system 10 comprises a tube 60, preferably rectilinear, which is to be set within the tank, preferably according to a vertical orientation, and is provided with one end 60A, the bottom one, which is open to allow entry of the solution of water and urea into the tube. The tube 60 defines an inner chamber 60C, positioned mobile inside which is a floating body 62, which is able to vary its position along the tube 60 as a function of the level of the solution within the tank. The top end 60B is coupled to a first optical element 64 and to a second optical element 66, which are connected to the emitter 2 and to the receiver 4, respectively. In particular, the two optical elements 64, 66 traverse an element 65 for closing the end 60B, giving out into the chamber 60C. The closing element 65 has the function of closing the end 60B of the tube 60 providing a vent 60D for the air and of forming a connection between the end 60B and the two optical elements 64, 66.

The beam of electromagnetic waves emitted by the emitter 2 is transmitted, through the optical element 64, within the tube 60.

Provided on the floating body 62 is a target surface 62A, facing the end 60B, which reflects the beam of electromagnetic waves back towards the end 60B itself. The beam of electromagnetic waves is then transmitted, through the optical element 66, to the receiver 4.

The control unit 20 is configured for determining the time taken by the beam of electromagnetic waves emitted by the emitter 2 to reach the receiver 4.

It will be noted that this time (also referred to as "time of flight") is variable as a function of the position of the float 62 along the tube 60 i.e., as a function of the level of the solution within the tank.

Consequently, the system 10 is able to obtain an indication of the level of the solution of water and urea in the tank by determining the "time of flight" of a beam of electromagnetic waves transmitted through the tube 60 in the modalities described above.

Advantageously, the system 10 can use for the aforesaid purpose the receiver 2, the emitter 4, and the control unit 20 already provided for measuring the concentration of urea.

Preferably, measurement of the concentration of urea and measurement of the level of the solution in the tank can be made in two distinct steps.

It should now be noted that the measurement of the concentration of urea in water constitutes the preferred application of the system described herein. In any case, the same system may be used for measuring concentrations of other solutions, for example mixtures, solvents, or percentages of bio-diesel in the diesel fuel. More in general, the system may be used for measuring a concentration of any solution for which the relation between the percent concentration and the refractive index is continuous and monotonic.

**Claims**

1. A system (10) for measuring the concentration of urea in solution with water, comprising:

   - an emitter (2) of a beam of electromagnetic waves;
   - a receiver (4) of said beam of electromagnetic waves;
   - a duct (6) provided for containing a solution of urea in water;
   - a first optical element (8) configured for optically coupling a first end (6A) of said duct (6) with said emitter (2);
   - a second optical element (12) configured for optically coupling a second end (6B), opposite to said first end (6A), of said duct (6) with said receiver (4), or else for optically coupling said first end (6A) of said duct (6) with said receiver (4), when said second end (6B) of said duct (6) is closed to reflect towards the first end (6A) of said duct (6) the electromagnetic waves emitted by the emitter (2) and coming from the first end itself; and
   - a control unit (20) configured for determining the concentration of urea in said solution as a function of a signal generated by said receiver, wherein said receiver is configured for detecting ,for a pre-set exposure time, the variation in unit time of the intensity of the beam of electromagnetic waves, and wherein said control unit is configured for determining the concentration of urea in said solution as a function of a variation in unit time of the intensity of the beam of electromagnetic waves received by said receiver.

2. The system according to claim 1, wherein said emitter (2) has a main direction of emission and is oriented so that said main direction of emission is oriented in a reference direction (R) of said system, and wherein said duct (6) has a pre-set length (L) and extends along a curvilinear path so as to present a dimension (H) in said main direction that is smaller than said pre-set length (L).

3. The system according to claim 2, wherein said duct (6) extends along a spiral or helical path.

4. The system according to any one of the preceding claims, wherein said duct (6') having said second end (6B) closed is rectilinear.

5. The system according to any one of the preceding claims, wherein said duct (6) is constituted by a tube of circular cross section.

6. The system according to any one of the preceding claims, wherein said duct (6) is made of a transparent material, preferably Teflon.

7. The system according to any one of the preceding claims, wherein said duct (6) has at least an inlet (6C) and an outlet (6D) for said solution, which are located in intermediate positions between said first and second ends (6A, 6B).

8. The system according to any one of the preceding claims, comprising a support (14) on which said emitter (2) and said receiver (4), said duct (6), and said first and second optical elements (8, 12) are mounted, wherein said support (14) has a first side and a second side, opposite to one another, wherein said duct (6) is mounted on said first side of said support (14) and said emitter (2) and said receiver (4) are mounted on said second side of said support (14), and wherein said first and second optical elements (8, 12) extend through said support (14) passing from said first side to said second side so as to connect optically said duct (6) to said emitter (2) and said receiver (4).

9. The system according to claim 2, wherein said first and second optical elements (8, 12) have cylindrical bodies and are inserted through said first and second ends (6A, 6B) of said duct (6), respectively, both being oriented with their own axis parallel to said reference direction (R), or else are both inserted through said first end of said duct and are parallel to one another.

10. The system according to any one of the preceding claims, wherein said duct has said closed second end (6B), further comprising:

    - a tube (60) having a first end (60A), which is open, for inlet of the solution into the tube and comprising a floating body (62), mobile along the tube as a function of a level of the solution within the tube (60);
    - a first optical element (64) configured for optically coupling a second end (60B) of the tube (60), opposite to said first end (60A), with said emitter (2); and
    - a second optical element (66) configured for optically coupling the second end (60B) of the tube (60) with said receiver (4),

    wherein the control unit (20) is configured for determining a level of the solution of water and urea as a function of a signal indicating a time of flight of a beam of electromagnetic waves that is emitted by the emitter (2), is reflected by the floating body (62), and is received by the receiver (4).

**Patentansprüche**

1. Ein System (10) zur Messung der Konzentration von Harnstoff in Lösung mit Wasser, umfassend:

    - einen Sender (2) eines Strahls elektromagnetischer Wellen;
    - einen Empfänger (4) besagten Strahls elektromagnetischer Wellen;
    - einen Kanal (6), der zur Aufnahme einer Lösung von Harnstoff in Wasser vorgesehen ist;
    - ein erstes optisches Element (8), das konfiguriert ist, um ein erstes Ende (6A) besagten Kanals (6) optisch mit besagtem Sender (2) zu koppeln;
    - ein zweites optisches Element (12), das konfiguriert ist, um ein zweites Ende (6B), gegenüberliegend zu besagtem ersten Ende (6A), besagten Kanals (6) optisch mit besagtem Empfänger (4) zu koppeln, oder alternativ besagtes erste Ende (6A) besagten Kanals (6) optisch mit besagtem Empfänger (4) zu koppeln, wenn besagtes zweite Ende (6B) besagten Kanals (6) geschlossen ist, um die vom Sender (2) emittierten und vom ersten Ende selbst kommenden elektromagnetischen Wellen zum ersten Ende (6A) besagten Kanals (6) zurück zu reflektieren;

und

    - eine Steuereinheit (20), die konfiguriert ist, um die Konzentration von Harnstoff in besagter Lösung als Funktion eines von besagtem Empfänger erzeugten Signals zu bestimmen, wobei besagter Empfänger konfiguriert ist, um für eine voreingestellte Belichtungszeit die Änderung pro Zeiteinheit der Intensität des Strahls elektromagnetischer Wellen zu erfassen, und wobei besagte Steuereinheit konfiguriert ist, um die Konzentration von Harnstoff in besagter Lösung als Funktion einer Änderung pro Zeiteinheit der Intensität des von besagtem Empfänger empfangenen Strahls elektromagnetischer Wellen zu bestimmen.

2. Das System nach Anspruch 1, wobei besagter Sender (2) eine Hauptemissionsrichtung aufweist und so orientiert ist, dass besagte Hauptemissionsrichtung in eine Referenzrichtung (R) besagten Systems orientiert ist, und wobei besagter Kanal (6) eine voreingestellte Länge (L) aufweist und sich entlang eines krummlinigen Pfades erstreckt, um eine Abmessung (H) in besagter Hauptrichtung aufzuweisen, die kleiner ist als besagte voreingestellte Länge (L).

3. Das System nach Anspruch 2, wobei besagter Kanal (6) sich entlang eines spiralförmigen oder schraubenförmigen Pfades erstreckt.

4. Das System nach einem der vorhergehenden Ansprüche, wobei besagter Kanal (6'), der besagtes geschlossenes zweites Ende (6B) aufweist, geradlinig ist.

5. Das System nach einem der vorhergehenden Ansprüche, wobei besagter Kanal (6) durch ein Rohr mit kreisförmigem Querschnitt gebildet ist.

6. Das System nach einem der vorhergehenden Ansprüche, wobei besagter Kanal (6) aus einem transparenten Material, vorzugsweise Teflon, hergestellt ist.

7. Das System nach einem der vorhergehenden Ansprüche, wobei besagter Kanal (6) mindestens einen Einlass (6C) und einen Auslass (6D) für besagte Lösung aufweist, die sich in Zwischenpositionen zwischen besagten ersten und zweiten Enden (6A, 6B) befinden.

8. Das System nach einem der vorhergehenden Ansprüche, umfassend einen Träger (14), auf dem besagter Sender (2) und besagter Empfänger (4), besagter Kanal (6) und besagte ersten und zweiten optischen Elemente (8, 12) montiert sind, wobei besagter Träger (14) eine erste Seite und eine zweite Seite, einander gegenüberliegend, aufweist, wobei besagter Kanal (6) auf besagter ersten Seite besagten Trägers (14) montiert ist und besagter Sender (2) und besagter Empfänger (4) auf besagter zweiten Seite besagten Trägers (14) montiert sind, und wobei besagte ersten und zweiten optischen Elemente (8, 12) sich durch besagten Träger (14) hindurch erstrecken, von besagter ersten Seite zu besagter zweiten Seite verlaufend, um besagten Kanal (6) optisch mit besagtem Sender (2) und besagtem Empfänger (4) zu verbinden.

9. Das System nach Anspruch 2, wobei besagte ersten und zweiten optischen Elemente (8, 12) zylindrische Körper aufweisen und durch besagte ersten und zweiten Enden (6A, 6B) besagten Kanals (6) eingeführt sind, jeweils mit ihrer eigenen Achse parallel zu besagter Referenzrichtung (R) orientiert, oder alternativ beide durch besagtes erste Ende besagten Kanals eingeführt sind und zueinander parallel verlaufen.

10. Das System nach einem der vorhergehenden Ansprüche, wobei besagter Kanal besagtes geschlossenes zweites Ende (6B) aufweist, ferner umfassend:

- ein Rohr (60) mit einem ersten Ende (60A), das offen ist, zum Einlass der Lösung in das Rohr und umfassend einen Schwimmkörper (62), der entlang des Rohrs als Funktion eines Füllstands der Lösung innerhalb des Rohrs (60) beweglich ist;
- ein erstes optisches Element (64), das konfiguriert ist, um ein zweites Ende (60B) des Rohrs (60), gegenüberliegend zu besagtem ersten Ende (60A), optisch mit besagtem Sender (2) zu koppeln; und
- ein zweites optisches Element (66), das konfiguriert ist, um das zweite Ende (60B) des Rohrs (60) optisch mit besagtem Empfänger (4) zu koppeln,

wobei die Steuereinheit (20) konfiguriert ist, um einen Füllstand der Lösung aus Wasser und Harnstoff als Funktion eines Signals zu bestimmen, das eine Laufzeit eines Strahls elektromagnetischer Wellen anzeigt, der vom Sender (2) emittiert wird, vom Schwimmkörper (62) reflektiert wird und vom Empfänger (4) empfangen wird.

## Revendications

1. Un système (10) pour mesurer la concentration d'urée en solution avec de l'eau, comprenant :

- un émetteur (2) d'un faisceau d'ondes électromagnétiques ;
- un récepteur (4) dudit faisceau d'ondes électromagnétiques ;
- un conduit (6) prévu pour contenir une solution d'urée dans l'eau ;
- un premier élément optique (8) configuré pour coupler optiquement une première extrémité (6A) dudit conduit (6) avec ledit émetteur (2) ;
- un deuxième élément optique (12) configuré pour coupler optiquement une deuxième extrémité (6B), opposée à ladite première extrémité (6A), dudit conduit (6) avec ledit récepteur (4), ou bien pour coupler optiquement ladite première extrémité (6A) dudit conduit (6) avec ledit récepteur (4), lorsque ladite deuxième extrémité (6B) dudit conduit (6) est fermée pour réfléchir vers la première extrémité (6A) dudit conduit (6) les ondes électromagnétiques émises par l'émetteur (2) et provenant de la première extrémité elle-même ; et
- une unité de commande (20) configurée pour déterminer la concentration d'urée dans ladite solution en fonction d'un signal généré par ledit récepteur, dans lequel ledit récepteur est configuré pour détecter, pendant un temps d'exposition prédéfini, la variation par unité de temps de l'intensité du faisceau d'ondes électromagnétiques, et dans lequel ladite unité de commande est configurée pour déterminer la concentration d'urée dans ladite solution en fonction d'une variation par unité de temps de l'intensité du faisceau d'ondes électromagnétiques reçu par ledit récepteur.

2. Le système selon la revendication 1, dans lequel ledit émetteur (2) a une direction principale d'émission et est orienté de sorte que ladite direction principale d'émission est orientée dans une direction de référence (R) dudit système, et dans lequel ledit conduit (6) a une longueur prédéfinie (L) et s'étend le long d'un trajet curviligne de manière à présenter une dimension (H) dans ladite direction principale qui est inférieure à ladite longueur prédéfinie (L).

3. Le système selon la revendication 2, dans lequel ledit conduit (6) s'étend le long d'un trajet en spirale ou hélicoïdal.

4. Le système selon l'une quelconque des revendications précédentes, dans lequel ledit conduit (6') ayant ladite deuxième extrémité (6B) fermée est rectiligne.

5. Le système selon l'une quelconque des revendications précédentes, dans lequel ledit conduit (6) est constitué d'un tube de section circulaire.

6. Le système selon l'une quelconque des revendications précédentes, dans lequel ledit conduit (6) est réalisé en un matériau transparent, de préférence du Téflon.

7. Le système selon l'une quelconque des revendications précédentes, dans lequel ledit conduit (6) comporte au moins une entrée (6C) et une sortie (6D) pour ladite solution, qui sont situées en des positions intermédiaires entre lesdites première et deuxième extrémités (6A, 6B).

8. Le système selon l'une quelconque des revendications précédentes, comprenant un support (14) sur lequel sont montés ledit émetteur (2) et ledit récepteur (4), ledit conduit (6), et lesdits premier et deuxième éléments optiques (8, 12), dans lequel ledit support (14) a un premier côté et un deuxième côté, opposés l'un à l'autre, dans lequel ledit conduit (6) est monté sur ledit premier côté dudit support (14) et ledit émetteur (2) et ledit récepteur (4) sont montés sur ledit deuxième côté dudit support (14), et dans lequel lesdits premier et deuxième éléments optiques (8, 12) s'étendent à travers ledit support (14) en passant dudit premier côté audit deuxième côté de manière à connecter optiquement ledit conduit (6) audit émetteur (2) et audit récepteur (4).

9. Le système selon la revendication 2, dans lequel lesdits premier et deuxième éléments optiques (8, 12) ont des corps cylindriques et sont insérés à travers lesdites première et deuxième extrémités (6A, 6B) dudit conduit (6), respectivement, les deux étant orientés avec leur propre axe parallèle à ladite direction de référence (R), ou bien sont tous deux insérés à travers ladite première extrémité dudit conduit et sont parallèles l'un à l'autre.

10. Le système selon l'une quelconque des revendications précédentes, dans lequel ledit conduit a ladite deuxième extrémité fermée (6B), comprenant en outre :

   - un tube (60) ayant une première extrémité (60A), qui est ouverte, pour l'entrée de la solution dans le tube et comprenant un corps flottant (62), mobile le long du tube en fonction d'un niveau de la solution à l'intérieur du tube (60) ;
   - un premier élément optique (64) configuré pour coupler optiquement une deuxième extrémité (60B) du tube (60), opposée à ladite première extrémité (60A), avec ledit émetteur (2) ; et
   - un deuxième élément optique (66) configuré pour coupler optiquement la deuxième extrémité (60B) du tube (60) avec ledit récepteur (4),

dans lequel l'unité de commande (20) est configurée pour déterminer un niveau de la solution d'eau et d'urée en fonction d'un signal indiquant un temps de vol d'un faisceau d'ondes électromagnétiques qui est émis par l'émetteur (2), est réfléchi par le corps flottant (62), et est reçu par le récepteur (4).

## FIG. 1

## FIG. 2

# FIG. 3

# FIG. 4

FIG. 5

EP 4 019 939 B1

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012170743 A1
- US 3513319 A
- US 2002102183 A1
- EP 2058682 A1
- WO 2008107336 A1